# EUROPEAN PATENT APPLICATION

(11) **EP 4 649 842 A1**
(43) Date of publication of application: **19.11.2025**
(21) Application number: 25177251.3
(22) Date of filing: 19.05.2025
(51) Int. Cl.: A24F 40/44

(54) **ATOMIZING CORE, ATOMIZER, AND ELECTRONIC ATOMIZATION DEVICE**

(30) Priority: 17.05.2024 CN 202421087577 U
(71) Applicant: Shenzhen Smoore Technology Limited, Shenzhen, Guangdong 518102 (CN)
(72) Inventor: ZHU, Haoliang, Shenzhen (CN); DU, Jiajun, Shenzhen (CN); LIN, Yan, Shenzhen (CN); ZHOU, Yu, Shenzhen (CN)
(74) Representative: Winter, Brandl - Partnerschaft mbB

(57) **Abstract**

The present disclosure relates to an atomizing core, an atomizer, and an electronic atomization device. The atomizing core is configured to heat an aerosol-forming medium to form an aerosol, where the atomizing core includes: a fluid inlet for the aerosol-forming medium to enter the atomizing core; and an atomization assembly, configured to heat and atomize the aerosol-forming medium; The atomization assembly includes a mounting member and a heating element. The atomizing core further includes a conductive assembly including a first conductive member and a second conductive member. The first conductive member is in fluid communication with the mounting member, and is configured to conduct the aerosol-forming medium, which is entered from the fluid inlet, to the mounting member. The second conductive member is in fluid communication with the mounting member, and a limiting portion is provided between the second conductive member and the first conductive member. The fluid conduction capability between the first conductive member and the second conductive member is worse than the fluid conduction capability between the mounting member and the first conductive member. According to the atomizing core, the problem of liquid leakage caused by saturation of the mounting member can be resolved.

## Description

### TECHNICAL FIELD

The present application relates to the field of atomization technologies, and in particular, to an atomizing core, an atomizer, and an electronic atomization device.

### BACKGROUND

An electronic atomization device is a device for forming an aerosol.

In a related technology, an atomizer includes a liquid storage cavity and an atomizing core arranged in the liquid storage cavity. The atomizing core is provided with a conductive member and an atomization assembly. An aerosol-forming medium in the liquid storage cavity may flow to the conductive member. The aerosol-forming medium is guided to the atomization assembly by using the conductive member, and then the aerosol-forming medium is heated and atomized by the atomization assembly. The atomizer is prone to liquid leakage caused by excessively rapid drainage from the liquid storage cavity.

### SUMMARY

The technical problem to be resolved by the present disclosure is to provide an atomizing core, an atomizer, and an electronic atomization device.

A technical solution adopted by the present disclosure to resolve the technical problem thereof is as follows. An atomizing core is disclosed, for heating an aerosol-forming medium to form an aerosol, where the atomizing core includes:
a fluid inlet for the aerosol-forming medium to enter the atomizing core; and
an atomization assembly, configured to heat and atomize the aerosol-forming medium; the atomization assembly including a heating element and a mounting member that is capable of conducting the aerosol-forming medium; and
the atomizing core further includes a conductive assembly including a first conductive member and a second conductive member;
the first conductive member being in fluid communication with the mounting member, and being arranged to conduct the aerosol-forming medium, which is entered from the fluid inlet, to the mounting member; and
the second conductive member being in fluid communication with the mounting member, and a limiting portion being provided between the second conductive member and the first conductive member, the fluid conduction capability between the first conductive member and the second conductive member being worse than the fluid conduction capability between the mounting member and the first conductive member.

**In** some embodiments, the limiting portion includes a gap region separating the first conductive member from the second conductive member.

**In** some embodiments, the limiting portion includes a first connection portion configured to conduct the aerosol-forming medium and connected between the first conductive member and the second conductive member; and
the limiting portion further includes a concave portion formed on or around the first connection portion, to reduce the fluid conduction capability between the first conductive member and the second conductive member.

**In** some embodiments, the limiting portion includes a second connection portion connected between the first conductive member and the second conductive member; and the porosity of the second connection portion is smaller than the porosity of the mounting member and the porosity of the first conductive member.

**In** some embodiments, the first conductive member and/or the second conductive member are/is arranged on a peripheral side of the mounting member.

**In** some embodiments, the mounting member is arranged between the heating element, the first conductive member, and the second conductive member.

**In** some embodiments, the first conductive member is adjacent to one end of the mounting member, and the second conductive member is adjacent to the other end of the mounting member.

**In** some embodiments, the first conductive member, the mounting member, and the second conductive member jointly form a flow channel; and
the second conductive member is arranged at a downstream position in an airflow direction of the flow channel, and the first conductive member is arranged at an upstream position in the airflow direction of the flow channel.

**In** some embodiments, the atomizing core further includes an outer tube for mounting the atomization assembly, and a sealing ring blocking one end of the outer tube;
the sealing ring being provided with a central channel that connects the flow channel to the outside of the outer tube, and the sealing ring abutting against the end of the second conductive member far away from the mounting member.

In some embodiments, the atomization assembly further includes a mounting base arranged between the mounting member, the first conductive member, and the second conductive member;
a first liquid transfer port and a second liquid transfer port are provided in the mounting; the first liquid transfer port being arranged between the first conductive member and the mounting member; and the second liquid transfer port being arranged between the second conductive member and the mounting member.

The present disclosure further discloses an atomizer, including a liquid storage tank for storing an aerosol-forming medium, and the atomizer further including the foregoing atomizing core.

The present disclosure further discloses an electronic atomization device, including an electronic control assembly, and further including the foregoing atomizer; the electronic control assembly being electrically connected to the atomizer.

Through implementation of the present disclosure, the following beneficial effects are achieved: In the atomizing core, by designing the first conductive member, the second conductive member, and the limiting portion between the first conductive member and the second conductive member, when the mounting member is saturated, an extra amount of the aerosol-forming medium formed due to saturation is absorbed by using the second conductive member.

### BRIEF DESCRIPTION OF THE DRAWINGS

The present disclosure is further described below with reference to the accompanying drawings and embodiments. In the accompanying drawings:
FIG. 1 is a schematic diagram of an atomizing core according to some embodiments of the present disclosure;
FIG. 2 is a longitudinal cross-sectional view of an atomizing core according to Embodiment 1 of the present disclosure;
FIG. 3 is a longitudinal cross-sectional view of an atomizing core in a partially disassembled state according to Embodiment 1 of the present disclosure; in which a heating element is omitted;
FIG. 4 is a schematic structural diagram of a mounting base of an atomizing core according to some embodiments of the present disclosure;
FIG. 5 is an enlarged view of content selected by the circular box pointed by the arrow A in FIG. 3;
FIG. 6 is a partial structural diagram of a conductive assembly and an atomization assembly according to Embodiment 2 of the present disclosure; and
FIG. 7 is a partial structural diagram of a conductive assembly and an atomization assembly according to Embodiment 3 of the present disclosure.

### List of Reference Numerals:

atomizing core 1; airflow channel 11; air inlet end 111; air outlet end 112; flow channel 113; outer tube 12; fluid inlet 121; atomization assembly 13; mounting member 131; heating element 132; mounting base 133; first liquid transfer port 1331; second liquid transfer port 1332; conductive assembly 14; first conductive member 141; second conductive member 142; limiting portion 143; gap region 1431; first connection portion 1432; concave portion 1433; second connection portion 1434; base 15; sealing ring 16; central channel 161.

### DETAILED DESCRIPTION

In order to have a clearer understanding of the technical features, the objectives, and the effects of the present disclosure, specific implementations of the present disclosure are now illustrated in detail with reference to the accompanying drawings. In the following description, it should be understood that orientation or position relationships indicated by the terms such as "front", "rear", "upper", "lower", "left", "right", "longitudinal", "transverse", "vertical", "horizontal", "top", "bottom", "inner", "outer", "head", and "tail" are based on orientation or position relationships shown in the accompanying drawings and structures and operations in specific orientations, and are used only for ease of description of the technical solution, rather than indicating that the device or element referred to must have a particular orientation. Therefore, such terms should not be construed as a limitation on the present disclosure.

It should also be noted that, unless otherwise explicitly specified and limited, the terms "mount", "connect", "connection", "fix", and "arrange" should be understood in a broad sense. For example, a connection may be a fixed connection, a detachable connection, or an integral connection; or the connection may be a mechanical connection or an electrical connection; or the connection may be a direct connection, an indirect connection through an intermediate medium, internal communication between two elements, or an interaction relationship between two elements. When an element is referred to as being "above" or "below" another element, the element can be "directly" or "indirectly" located above the another element, or one or more intervening elements may also exist. The terms such as "first", "second", and "third" are used only for ease of description of the technical solution, and cannot be construed as indicating or implying relative importance or implying a quantity of indicated technical features. Therefore, features defined by "first", "second", and "third" may explicitly indicate or implicitly include one or more of the features. A person of ordinary skill in the art can understand specific meanings of the terms in the present disclosure according to specific situations.

In the following description, for the purpose of illustration rather than limitation, specific details such as a specific system structure and technology are proposed to thoroughly understand the present disclosure. However, it should be clear to a person skilled in the art that the present disclosure may also be implemented in other embodiments without these specific details. In other cases, detailed descriptions of well-known systems, apparatuses, circuits, and methods are omitted, so as not to obscure the description of the present disclosure with unnecessary details.

This application provides an electronic atomization device. The electronic atomization device may be configured to atomize an aerosol-forming medium. The electronic atomization device includes an atomizer and an electronic control module that are electrically connected to each other.

The atomizer is configured to store the aerosol-forming medium and heat and atomize the aerosol-forming medium to form an aerosol that can be smoked/inhaled by a user. The atomizer may specifically be applied to different fields such as medical care, cosmetology, and recreation inhalation. The aerosol-forming medium is a fluid. The fluid may be a liquid or a gas. The following describes the atomizer of this application by using an example in which the aerosol generated by the atomizer is inhaled by the user.

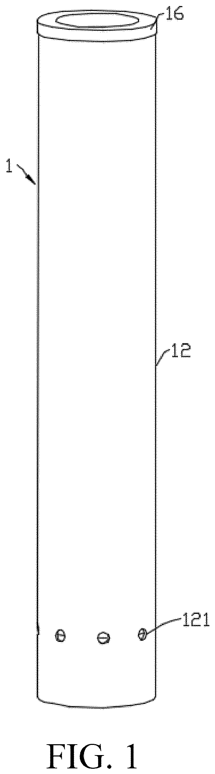

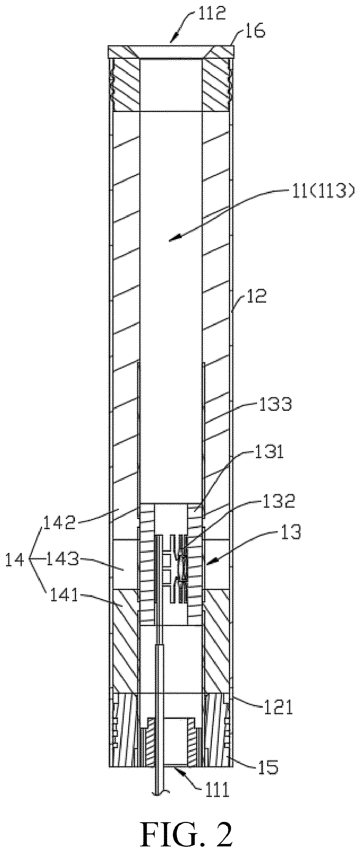

The atomizer may include a liquid storage cavity (not shown) configured to store the aerosol-forming medium, and an atomizing core 1 (refer to FIG. 1) in fluid communication with the liquid storage cavity. The aerosol-forming medium in the liquid storage cavity may flow to the atomizing core 1, and the aerosol-forming medium is heated by the atomizing core 1. Meanwhile, referring to FIG. 2, an airflow channel 11 is formed in the atomizing core 1, and external air may flow in from one end (an air inlet end 111) of the airflow channel 11, and flow out from the other end (an air outlet end 112) of the airflow channel 11, to take out the atomized aerosol-forming medium.

The electronic control module (not shown) may include a battery and a controller. The battery is configured to supply power to the atomizer. The controller is configured to control operation of the atomizer. For a specific structure of the controller and a control method, refer to related technologies. Details are not described herein. The electronic control module may further include other elements such as a battery holder and an airflow sensor. Details are not described herein.

In some embodiments, referring to FIG. 2, the atomizing core 1 may include an outer tube 12, a conductive assembly 14, and an atomization assembly 13. The outer tube 12 may be arranged in the liquid storage cavity, and at least one fluid inlet 121 in communication with the liquid storage cavity is arranged in the outer tube 12. The aerosol-forming medium may enter the outer tube 12 through the fluid inlet 121. Certainly, the fluid inlet 121 does not necessarily need to be formed in the outer tube 12, and may alternatively be arranged in another structure of the atomizing core 1. In other words, the outer tube 12 is not a necessary component of the atomizing core 1, as long as the aerosol-forming medium can enter the atomizing core 1. The conductive assembly 14 is configured to store and conduct the aerosol-forming medium, which may conduct the aerosol-forming medium to the atomization assembly 13. The atomization assembly 13 may absorb the aerosol-forming medium and heat and atomize the aerosol-forming medium.

As shown in FIG. 2, the atomization assembly 13 may include a mounting member 131 and a heating element 132 mounted on the mounting member 131. The mounting member 131 may conduct, to the heating element 132, the aerosol-forming medium conducted by the conductive assembly 14, and the aerosol-forming medium is heated by the heating element 132.

Referring to the structure of the conductive assembly 14, the conductive assembly 14 may include a first conductive member 141 and a second conductive member 142.

The first conductive member 141 may be configured to store and conduct the aerosol-forming medium, which is in fluid communication with the mounting member 131. Moreover, the first conductive member 141 is closer to the fluid inlet 121 than the second conductive member 142, and the aerosol-forming medium entered from the fluid inlet 121 may first flow through the first conductive member 141 to guide the aerosol-forming medium in a liquid storage tank to the mounting member 131. The "in fluid communication" may be understood as enabling fluid transmission therebetween.

The second conductive member 142 may also be configured to store and conduct the aerosol-forming medium, and is in fluid communication with the mounting member 131. However, a limiting portion 143 is provided between the second conductive member 142 and the first conductive member 141, so that the fluid conduction capability between the first conductive member 141 and the second conductive member 142 may be worse than the fluid conduction capability between the mounting member 131 and the first conductive member 141.

It may be understood that the foregoing fluid conduction capability is used for reflecting a degree of difficulty of fluid conduction between the first conductive member 141 and the second conductive member 142 and between the mounting member 131 and the first conductive member 141. In this application, through the design of the limiting portion 143, the fluid conduction capability between the first conductive member 141 and the second conductive member 142 is affected, so that the fluid conduction capability between the first conductive member 141 and the second conductive member 142 may be worse than the fluid conduction capability between the mounting member 131 and the first conductive member 141. At the same time, it should be noted that, different from conduction efficiency, the fluid conduction capability is only used to represent performance at a structural level, and an influence of an external factor thereon is not taken into account. The influence of the external factor is, for example, reduction in the conduction efficiency due to absorption of the aerosol-forming medium to saturation.

To ensure the taste of the electronic atomization device in the related technology, a ventilation channel that communicates the liquid storage cavity with the outside may generally be selected, so as to speed up drainage from the liquid storage cavity. However, due to a limited storage capacity of the conductive member, when the drainage from the liquid storage cavity is excessively fast, the conductive member is prone to saturation, and the aerosol-forming medium thereon may be squeezed into the mounting member 131. As a result, the aerosol-forming medium absorbed by the mounting member 131 is oversaturated, thereby causing liquid leakage. Especially when the electronic atomization device is left aside for a long time, liquid leakage is more likely to occur.

However, in this application, the electronic atomization device is provided with the first conductive member 141 and the second conductive member 142, where the first conductive member 141 is configured to guide the aerosol-forming medium in the liquid storage tank to the mounting member 131. Due to the existence of the limiting portion 143, the aerosol-forming medium in the liquid storage tank may not be easily conducted to the second conductive member 142. When efficiency at which the aerosol-forming medium is consumed by the heating element 132 is equivalent to efficiency at which the first conductive member 141 conducts the aerosol-forming medium to the mounting member 131, the aerosol-forming medium may not be conducted to the second conductive member 142, or only a minimal amount may be conducted to the second conductive member 142. However, when the aerosol-forming medium at the mounting member 131 is saturated and is about to leak, the aerosol-forming medium may be actively conducted to the second conductive member 142, and part of the aerosol-forming medium on the mounting member 131 and/or the first conductive member 141 may be absorbed by the second conductive member 142, thereby resolving the problem of liquid leakage.

The following specifically describes a specific structure and a connection relationship of the atomizing core 1 in some embodiments. For ease of description of the atomizing core 1 of this application, referring to FIG. 2 below, the side of the structure close to the air inlet end 111 of the atomizing core 1 is referred to as a bottom/bottom end, and the side of the structure far away from the air inlet end 111 of the atomizing core 1 is referred to as a top/top end.

### Embodiment 1

Referring to FIG. 2, the outer tube 12 may be cylindrical. Certainly, the outer tube 12 may alternative be in another shape, for example, a square tube, which is not limited herein. A peripheral side of the outer tube 12 is provided with a plurality of fluid inlets 121.

As shown in FIG. 2, the mounting member 131 includes a liquid absorbing surface and an atomization surface opposite to the liquid absorbing surface. The liquid absorbing surface is used to be in fluid communication with the first conductive member 141 and the second conductive member 142, to conduct the aerosol-forming medium. The aerosol-forming medium on the liquid absorbing surface may be conducted to the atomization surface, and the atomization surface is used to be connected to the heating element 132. The aerosol-forming medium conducted to the atomization surface is heated by the heating element 132.

Optionally, the mounting member 131 may be tubular, of which an outer side surface serves as the liquid absorbing surface and an inner side surface serves as the atomization surface.

The heating element 132 may include one or more heating circuits and electrode portions respectively arranged on two sides of each heating circuit along a length direction thereof. The heating circuit is a sheet-like structure and is arranged to be adhered to an inner peripheral side of the mounting member 131 in a curly manner. When a plurality of heating circuits are arranged, the plurality of heating circuits may be arranged at intervals along an axial direction of the mounting member 131. The electrode portions are configured to be connected to a power supply.

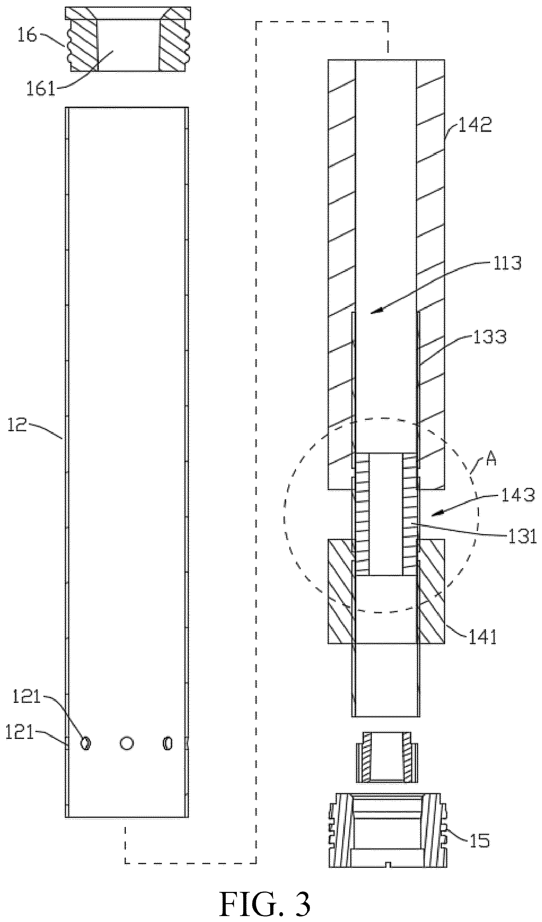

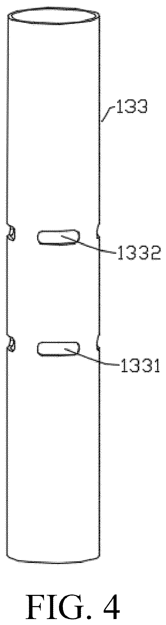

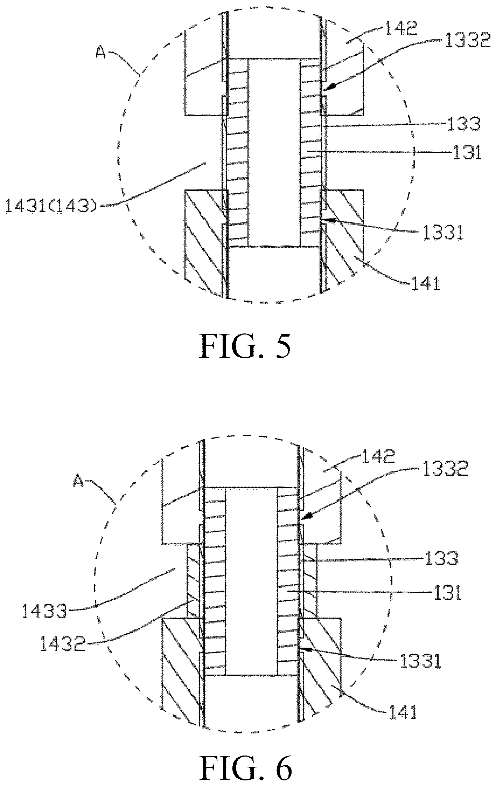

Referring to FIG. 3, the atomization assembly 13 may further include a tubular mounting base 133 configured to provide a basis for the mounting member 131 to be connected to the first conductive member 141 and the second conductive member 142. The mounting base 133 may sleeve the outer periphery of the mounting member 131. Referring to FIG. 4, a peripheral side of the mounting base 133 is further provided with a first liquid transfer port 1331 and a second liquid transfer port 1332 that are through and axially arranged at intervals. Referring to FIG. 5, the first liquid transfer port 1331 is arranged between the first conductive member 141 and the mounting member 131, to enable fluid communication between the first conductive member 141 and the mounting member 131, and the second liquid transfer port 1332 is arranged between the second conductive member 142 and the mounting member 131, to enable fluid communication between the second conductive member 142 and the mounting member 131.

Optionally, a plurality of first liquid transfer ports 1331 may be provided and arranged in a peripheral direction of the mounting base 133. Similarly, a plurality of second liquid transfer ports 1332 may also be provided and arranged in the peripheral direction of the mounting base 133.

The first conductive member 141 and the second conductive member 142 are made of a capillary material. The capillary material is a material that can implement fluid conduction. Optionally, the capillarity material may be a capillary tube material. The capillary tube material may optionally be a porous material. For example, the first conductive member 141 and the second conductive member 142 are porous ceramics. Certainly, the capillary tube material may alternatively be a sponge-like, fiber-like, or bubble-like material. For example, the first conductive member 141 and the second conductive member 142 are liquid-conducting cotton.

The mounting member 131, the first conductive member 141, and the second conductive member 142 may be made of a same material, or may be made of different materials, which is not specifically limited herein as long as fluid circulation can be achieved. Similarly, the pore diameters and porosity of the mounting member 131, the first conductive member 141, and the second conductive member 142 are not limited, as long as fluid circulation can be achieved.

Referring back to FIG. 2, the first conductive member 141 may be tubular and mounted in the outer tube 12. The outer diameter of the first conductive member 141 may be equivalent to the inner diameter of the outer tube 12, so that an outer peripheral side surface of the first conductive member 141 may be in contact with an inner peripheral side surface of the outer tube 12. An outer side surface of the first conductive member 141 may be at least partially arranged opposite to an orifice of the fluid inlet 121 in a radial direction of the outer tube 12, so as to facilitate liquid feeding.

Similarly, the second conductive member 142 may be tubular and mounted in the outer tube 12, and the second conductive member 142 is closer to the air outlet end 112 of the airflow channel 11 than the first conductive member 141. The outer diameter of the second conductive member 142 may be equivalent to the inner diameter of the outer tube 12, so that an outer peripheral side surface of the second conductive member 142 may be in contact with the inner peripheral side surface of the outer tube 12.

As shown in FIG. 3, the first conductive member 141 may be arranged to be in fluid communication with the bottom end of the mounting member 131. Optionally, the inner diameter of the first conductive member 141 may be equivalent to the outer diameter of the mounting member 131. The first conductive member 141 may sleeve an outer peripheral side of the mounting member 131, so that an inner peripheral side surface of the first conductive member 141 may be in contact with an outer peripheral side surface of the mounting member 131. During the assembly, the first conductive member 141 and the mounting member 131 are respectively adhered to inner and outer side surfaces of the mounting base 133, to ensure that a space between the first liquid transfer port 1331, the first conductive member 141, and the mounting member 131 is sealed, thereby preventing liquid leakage. Further optionally, the first conductive member 141 is inserted into the mounting member 131 from the bottom end of the mounting member 131. The outer peripheral side surface of the first conductive member 141 is in contact with the inner peripheral side surface of the mounting member 131.

Similarly, the second conductive member 142 may be arranged to be in fluid communication with the top end of the mounting member 131. The inner diameter of the second conductive member 142 may be equivalent to the outer diameter of the mounting member 131, so that an inner peripheral side surface of the second conductive member 142 may be in contact with the outer peripheral side surface of the mounting member 131. During the assembly, the second conductive member 142 and the mounting member 131 are respectively adhered to the inner and outer side surfaces of the mounting base 133, to ensure that a space between the second liquid transfer port 1332, the second conductive member 142, and the mounting member 131 is sealed, thereby preventing liquid leakage. Further optionally, the second conductive member 142 is inserted into the mounting member 131 from the top end of the mounting member 131. The outer peripheral side surface of the second conductive member 142 is in contact with the inner peripheral side surface of the mounting member 131.

In Embodiment 1, the second conductive member 142 and the first conductive member 141 are arranged at intervals along the axial direction of the mounting member 131, and there is a gap region 1431 therebetween. The gap region 1431 is used as the limiting portion 143, so that the fluid conduction capability between the first conductive member 141 and the second conductive member 142 is zero, and the aerosol-forming medium in a first external liquid storage tank needs to pass through the first conductive member 141 and the mounting member 131 in sequence to reach the second conductive member 142. However, when the aerosol-forming medium at the mounting member 131 is saturated and is about to leak, the second conductive member 142 may absorb the aerosol-forming medium oversaturated at the mounting member 131, thereby resolving the problem of liquid leakage caused by the saturation of the aerosol-forming medium at the mounting member 131.

Still referring to FIG. 3, a flow channel 113 may be jointly defined between the first conductive member 141, the second conductive member 142, and the mounting member 131. The flow channel 113 may be used as part of the airflow channel 11.

The second conductive member 142 is arranged at a downstream position in an airflow direction of the flow channel 113, and the first conductive member 141 is arranged at an upstream position in the airflow direction of the flow channel 113.

It should be noted here that, after airflow passes through the atomization assembly 13 to form an aerosol, the aerosol may flow in a direction from an inner periphery of the second conductive member 142 to the air outlet end 112. During the flowing, part of the aerosol may turn into condensate due to lower temperatures. In this case, since the second conductive member 142 is arranged at the downstream position in the airflow direction of the flow channel 113, the second conductive member 142 may absorb the condensate. In this way, when a degree of humidity of the mounting member 131 is less than that of the second conductive member 142 (for example, when the aerosol-forming medium in the liquid storage cavity is insufficient), the second conductive member 142 may re-transmit the absorbed aerosol-forming medium (including absorbing the aerosol-forming medium overflowing from the mounting member 131 and the condensate) to the mounting member 131, so that when an amount of liquid stored in the liquid storage cavity is definite, the use time of the electronic atomization device can be prolonged (that is, a quantity of puffs may be increased), and an influence of the condensate on the puffing taste of the user may also be prevented. Referring to FIG. 2, the atomizing core 1 may further include a base 15 mounted in the outer tube 12 and blocking a bottom opening portion of a shell. The base 15 may be a plastic member. During the assembly, the base 15 may be mounted in the outer tube 12 by riveting. In this case, the first conductive member 141 may be arranged at the top of the base 15. A mounting bracket may be inserted into the base 15, so as to fix the mounting bracket in the outer tube 12.

The base 15 may be a tubular structure, the maximum outer diameter of which may be equivalent to the inner diameter of the outer tube 12, and is used to seal a position of an inner peripheral edge of the outer tube 12. At the same time, an inner periphery of the base 15 may be in communication with the inside and the outside of the shell, and the airflow may pass through the inside of the base 15 and flow to the first conductive member 141.

Still referring to FIG. 2, the atomizing core 1 may further include a sealing ring 16 mounted in the outer tube 12 and blocking a top opening portion of the shell. During the assembly, after the second conductive member 142 is loaded into the outer tube 12, the second conductive member 142 is pressed, by using the sealing ring 16, down to be opposite to the second liquid transfer port 1332 of the mounting base 133. After the assembly, the top of the second conductive member 142 abuts against the sealing ring 16. The sealing ring 16 may be a tubular structure, optionally a silicone member, and is configured to seal the position of the inner peripheral edge of the outer tube 12. At the same time, the sealing ring 16 is provided with a central channel 161 that may be in communication with the inside and the outside of the shell. The airflow may pass through the inside of the sealing ring 16 and flow out to the outside of the shell.

### Embodiment 2

Referring to FIG. 6, the atomizing core 1 in this embodiment is modified based on Embodiment 1. The main modification is a change in the limiting portion 143. The following describes a structure, which is different from that in Embodiment 1, of the atomizing core 1 in Embodiment 2. For the remaining unmentioned structures of the atomizing core 1, please refer to Embodiment 1. Details are not described herein again.

In Embodiment 2, the limiting portion 143 includes a first connection portion 1432 configured to conduct the aerosol-forming medium and connected between the first conductive member 141 and the second conductive member 142. The limiting portion 143 further includes a concave portion 1433 formed on or around the first connection portion 1432, to reduce the fluid conduction capability between the first conductive member 141 and the second conductive member 142.

It may be understood that the concave portion 1433 is configured to reduce a fluid conduction area between the first conductive member 141 and the second conductive member 142, so as to achieve a purpose of reducing the fluid conduction capability between the first conductive member 141 and the second conductive member 142.

The first connection portion 1432 may be tubular, the outer diameter of which is smaller than the outer diameter of the first conductive member 141 and the outer diameter of the second conductive member 142. The concave portion 1433 is formed at an outer peripheral side of the first connection portion 1432. Certainly, the forming manner of the concave portion 1433 is not specifically limited herein. Further optionally, the first connection portion 1432 is tubular, the inner diameter of which is greater than the inner diameter of the first conductive member 141 and the inner diameter of the second conductive member 142. The concave portion 1433 is formed at an inner peripheral side of the first connection portion 1432 and between the first connection portion 1432 and the mounting member 131. Further optionally, the first connection portion 1432 is slotted to form the concave portion 1433.

The fluid conduction capability (such as the porosity) of the first connection portion 1432, the size of the concave portion 1433, and a quantity of the concave portion 1433 may be set according to a product requirement, provided that the fluid conduction capability between the first conductive member 141 and the second conductive member 142 is worse than the fluid conduction capability between the mounting member 131 and the first conductive member 141.

### Embodiment 3

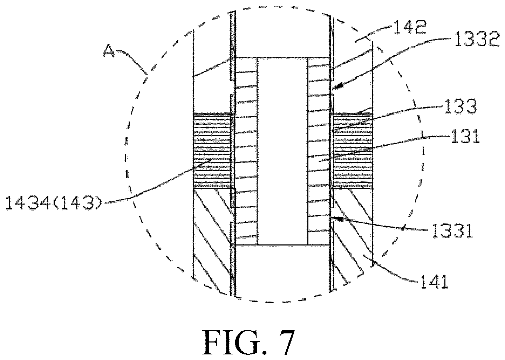

Referring to FIG. 7, the atomizing core 1 in this embodiment is modified based on Embodiment 1. The main modification is a change in the limiting portion 143. The following describes a structure, which is different from that in Embodiment 1, of the atomizing core 1 in Embodiment 3. For the remaining unmentioned structures of the atomizing core 1, please refer to Embodiment 1. Details are not described herein again.

In Embodiment 3, the limiting portion 143 includes a second connection portion 1434 connected between the first conductive member 141 and the second conductive member 142.

The second connection portion 1434 may be a capillary structure, that is, made of a capillary tube material, or may be a structure manufactured with a capillary tube. The porosity of the second connection portion 1434 is greater than zero and is less than the porosity of the mounting member 131 and the porosity of the first conductive member 141, so that the aerosol-forming medium on the first conductive member 141 is preferentially conducted to the mounting member 131.

The second connection portion 1434 may alternatively be a closed structure. That is, the porosity thereon is zero, and only the aerosol-forming medium on the mounting member 131 can be conducted to the second conductive member 142.

Secondly, the second connection portion 1434 may be tubular, the outer diameter of which may be equivalent to the outer diameter of the first conductive member 141 and the outer diameter of the second conductive member 142. Certainly, the size may be adjusted according to an actual requirement, which is not specifically limited herein.

In summary, in the atomizing core of this application, by designing the first conductive member 141, the second conductive member 142, and the limiting portion 143 between the first conductive member 141 and the second conductive member 142, generally, the aerosol-forming medium entered from the fluid inlet 121 is conducted to the mounting member 131 by using the first conductive member 141, and when the mounting member 131 is saturated, an extra amount of the aerosol-forming medium formed due to saturation is absorbed by using the second conductive member 142, which effectively alleviates/resolves the problem of liquid leakage caused by the saturation of the mounting member 131.

Secondly, the second conductive member 142 may also prolong the use time by recovering the condensate and absorbing the aerosol-forming medium overflowing from the mounting member 131.

## Claims

1. An atomizing core, for heating an aerosol-forming medium to form an aerosol, **characterized in that** the atomizing core comprises:
a fluid inlet (121) for the aerosol-forming medium to enter the atomizing core; and
an atomization assembly (13), configured to heat and atomize the aerosol-forming medium; the atomization assembly (13) comprising a heating element (132) and a mounting member (131) that is capable of conducting the aerosol-forming medium;
wherein the atomizing core further comprises a conductive assembly (14) comprising a first conductive member (141) and a second conductive member (142);
the first conductive member (141) is in fluid communication with the mounting member (131), and is arranged to conduct the aerosol-forming medium, which is entered from the fluid inlet (121), to the mounting member (131); and
the second conductive member (142) is in fluid communication with the mounting member (131), and a limiting portion (143) is provided between the second conductive member (142) and the first conductive member (141), the fluid conduction capability between the first conductive member (141) and the second conductive member (142) is worse than the fluid conduction capability between the mounting member (131) and the first conductive member (141).

2. The atomizing core of claim 1, wherein the limiting portion (143) comprises a gap region (1431) separating the first conductive member (141) from the second conductive member (142).

3. The atomizing core of claim 1, wherein the limiting portion (143) comprises a first connection portion (1432) configured to conduct the aerosol-forming medium and connected between the first conductive member (141) and the second conductive member (142); and
the limiting portion (143) further comprises a concave portion (1433) formed on or around the first connection portion (1432), to reduce the fluid conduction capability between the first conductive member (141) and the second conductive member (142).

4. The atomizing core of claim 1, wherein the limiting portion (143) comprises a second connection portion (1434) connected between the first conductive member (141) and the second conductive member (142); and the porosity of the second connection portion (1434) is smaller than the porosity of the mounting member (131) and the porosity of the first conductive member (141).

5. The atomizing core of claim 1, wherein the first conductive member (141) and/or the second conductive member (142) are/is arranged on a peripheral side of the mounting member (131).

6. The atomizing core of claim 1, wherein the mounting member (131) is arranged between the heating element (132), the first conductive member (141), and the second conductive member (142).

7. The atomizing core of claim 1, wherein the first conductive member (141) is adjacent to one end of the mounting member (131), and the second conductive member (142) is adjacent to the other end of the mounting member (131).

8. The atomizing core of claim 1, wherein the first conductive member (141), the mounting member (131), and the second conductive member (142) jointly form a flow channel (113); and
the second conductive member (142) is arranged at a downstream position in an airflow direction of the flow channel (113), and the first conductive member (141) is arranged at an upstream position in the airflow direction of the flow channel (113).

9. An atomizer, comprising a liquid storage tank for storing an aerosol-forming medium, wherein the atomizer further comprises the atomizing core of any one of claims 1 to 8.

10. An electronic atomization device, comprising an electronic control assembly, and further comprising the atomizer of claim 9; the electronic control assembly being electrically connected to the atomizer.
